# EUROPEAN PATENT APPLICATION

(11) **EP 3 881 821 A1**
(43) Date of publication of application: **22.09.2021**
(21) Application number: 20164213.9
(22) Date of filing: 19.03.2020
(51) Int. Cl.: A61K 8/55, A61Q 19/08

(54) **PHOSPHINIC PEPTIDE DERIVATIVES FOR USE AS MMP-3 INHIBITORS**

(71) Applicant: DSM IP Assets B.V., 6411 TE Heerlen (NL)
(72) Inventor: GLAUS, Florian David, 4303 Kaiseraugst (CH); JACKSON, Eileen, 4303 Kaiseraugst (CH); WANDELER, Eliane Ursula, 4303 Kaiseraugst (CH); WIKSTROEM, Peter, 4303 Kaiseraugst (CH)
(74) Representative: Berg, Katja

(57) **Abstract**

The present invention relates to the use of certain MMP-3 inhibitors, in particular in a cosmetic composition for inhibiting the degradation of the molecules of the basement membrane. In addition the present invention relates to the use of certain compounds as inhibitors of Matrix Metalloprotease-3 (MMP-3) as well as for the prevention and/or treatment of ailments associated with MMP-3.

## Description

The present invention relates to the use of certain MMP-3 inhibitors, in particular in a cosmetic composition for inhibiting the degradation of the molecules of the basement membrane. In addition the present invention relates to the use of certain compounds as inhibitors of Matrix Metalloprotease-3 (MMP-3) as well as for the prevention and/or treatment of ailments associated with MMP-3.

The basement membrane (BM) on the dermal-epidermal join has several functions, of which the most obvious function is close joining of the epidermis to the dermis, and in this way ensures mechanically stable cohesion of the two layers of tissue. The "polarity" and the construction of the epidermis are also influenced by the basement membrane, and at the same time the BM is a clear demarcation between the epidermis and dermis. It is assumed that the BM initiates epidermal differentiation of keratinocytes and maintains the proliferative state of the basal cell layer.

A further important function of the BM is correct communication between the epidermal and dermal cells. Since the epidermis and the dermis do not function independently of one another, normal skin homeostasis requires regular passage of biochemical signals in both directions between the two cell types. In this context, the BM has the important function of acting as an active filter and of passing on the signal molecules or just blocking them as required. Correctly functioning epidermal-dermal communication via the BM is thus essential for skin health.

The major molecular constituents of basement membranes are collagen-IV, laminin, and proteoglycans.

Type IV collagen is the main collagen component of the basement membrane. It is a network-forming collagen that functions as a barrier between tissue compartments, which facilitates transportation of nutrients and waste products though the dermal-epidermal junction. Collagen-IV is also of importance in wound healing.

Proteoglycans are natural macromolecules that are capable of restoring epidermal cells and intensifying the metabolism of connective tissue components, restoring the skin's functions, mechanical properties and physiological appearance.

Proteoglycans carry out the following activities within connective tissue: they increase cell proliferation and metabolism, they increase fatty acid turnover, they have anti-inflammatory activity, they accelerate the healing process, they increase the connection of cells with the extracellular components in their environment, they increase communication between cells, they have a structural function within connective tissue, they regulate the water content of connective tissue and its water retention capacity.

The laminins are a family of glycoproteins and are an important and biologically active part of the basal lamina, one of the layers of the basement membrane, influencing cell differentiation, migration, and adhesion.

Matrix metalloproteinases (MMPs) are a group of zinc-dependent extracellular proteinases, , which remodel the extracellular matrix (ECM). Based on their structure and substrate specificity they can be categorized into collagenases which degrade fibrillar collagen (MMP-1, -8 or 13), gelatinases which degrade base membrane collagen or any other form of denaturalized collagen (MMP-2 or -9), matrilysin that degrades the basement membrane (MMP-7,-26) metalloelastase, that degrades primarily elastin (MMP-12) and stromelysins which degrade basement membrane collagens, proteoglycans and matrix glycoproteins (MMP-3, -10 and -11).

MMP-3 is in particular known to readily degrade collagen-IV, proteoglycans and laminin. In addition, MMP-3 can also activate other MMPs such as MMP-1, MMP-7, and MMP-9, rendering MMP-3 crucial in connective tissue remodeling.

Thus, externally supplied active compounds which inhibit MMP-3 can protect collagen-IV, laminin and proteoglycans in the BM and are thus a highly attractive target to compensate age-related degenerations of the BM and also exhibit a preventive character by slowing down the decrease in the BM molecules such as in particular collagen-IV, laminin and proteoglycans.

Surprisingly it has been found that the compounds of formula (I) 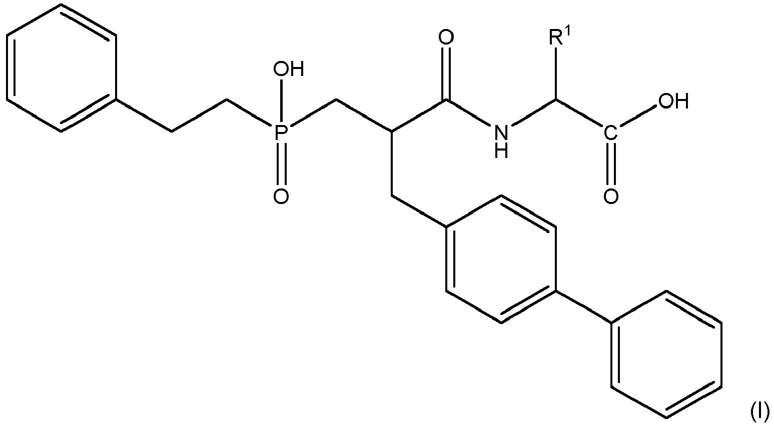 wherein
R¹ is a linear or branched C₁-C₈alkyl group are highly effective MMP-3 inhibitors at very low use levels and can thus be used to protect the degradation of the molecules of the basement membrane.

Thus, in a first aspect, the present invention relates to the use of a compound of formula (I) 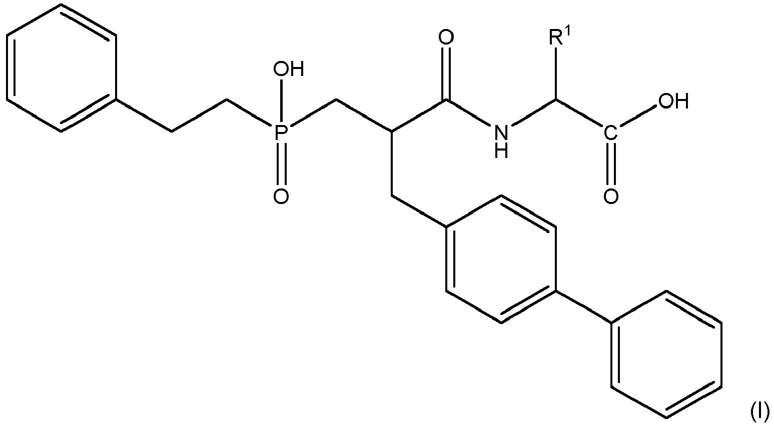 wherein
R¹ is a linear or branched C₁-C₁₀alkyl group
or a cosmetically acceptable salt thereof as cosmetic active ingredient for inhibiting the degradation of the molecules of the basement membrane

In another aspect, the present invention relates to the use of a compound of formula (I) as defined above or a cosmetically acceptable salt thereof as MMP-3 inhibitor as well as for the prevention and/or treatment of ailments associated with MMP-3.

In further aspect, the present invention relates to a method of inhibiting the degradation of molecules of the basement membrane, which method comprises topically administering to a person in need of such inhibition a composition containing an effective amount of at least one compound of formula (I) or a cosmetically acceptable salt thereof as defined above.

Examples of C₁-C₁₀alkyl groups are unbranched C₁-C₁₀alkyl or branched C₃-C₁₀alkyl groups such as methyl, ethyl, n-propyl, 1-methylethyl, n-butyl, 1-methylpropyl, 2-methylpropyl, 1,1-dimethylethyl, n-pentyl, 1-methylbutyl, 2-methylbutyl, 3-methylbutyl, 2,2-dimethylpropyl, 1-ethylpropyl, n-hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, and 1-ethyl-2-methylpropyl groups. Preferred C₁-C₁₀alkyl groups in all embodiments of the present invention are linear or branched C₁-C₆alkyl groups, even more preferably linear or branched C₁-C₄alkyl groups. Most preferably, in all embodiments of the present invention R¹ is selected from the group consisting of methyl, butyl and sec-butyl.

It is well understood, that the present invention encompasses the compounds of formula (I) as optically pure isomers such as e.g. as pure enantiomers or stereoisomers as well as mixtures of different isomers such as e.g. as racemates, or mixtures of diastereoisonmers.

The term 'or a cosmetically acceptable salt thereof refers to compounds of formula (I) in the form of a salt such as in the form of a salt formed by the addition of a base such as an alkali or earth alkaline salt, in particular lithium, sodium, potassium, magnesium or calcium salts. Most preferred according to the present invention are the compounds as such or in the form of their lithium, sodium or potassium salts. Most preferred are the compounds as such or in the form of their sodium salts. Such salts are easily prepared by a person skilled in the art. It is well understood, that the salt can also be formed within a composition according to the present invention, e.g. in dependence of the pH thereof and a cation present therein which is e.g. introduced into the composition in the form of a base such as sodium hydroxide.

Most preferred compounds of formula (I) are outlined in table 1:

**Table 1**

| **Structure** | | **Name** |
|---|---|---|
| 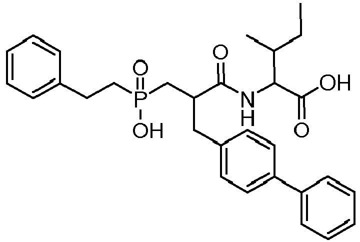 | (I)-a | 2-(3-([1,1'-biphenyl]-4-yl)-2-((hydroxy(phenethyl)phosphoryl)methyl)pro panamido)-3-methylpentanoic acid |
| 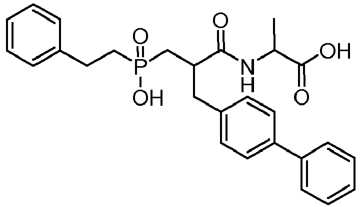 | (I)-b | (3-([1,1'-biphenyl]-4-yl)-2-((hydroxy(phenethyl)phosphoryl) methyl) pro panoyl)alanine |
| 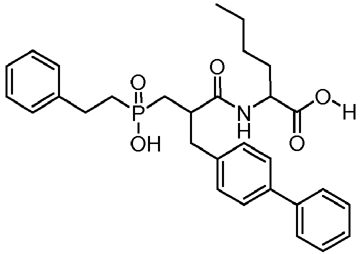 | (I)-c | 2-(3-([1,1'-biphenyl]-4-yl)-2-((hydroxy(phenethyl)phosphoryl)methyl)pro panamido)hexanoic acid |

Particularly advantageous stereoisomers according to the present invention are the ones listed in table 2:

**Table 2**

| **Name** | |
|---|---|
| ((2S)-3-([1,1'-biphenyl]-4-yl)-2-((hydroxy(phenethyl)phosphoryl)methyl)propanoyl)-L-isoleucine | (I)-A |
| ((2S)-3-([1,1'-biphenyl]-4-yl)-2-((hydroxy(phenethyl)phosphoryl)methyl)propanoyl)-L-alanine | (I)-B |
| ((2S)-3-([1,1'-biphenyl]-4-yl)-2-((hydroxy(phenethyl)phosphoryl)methyl)propanoyl)-L-norleucine | (I)-C |

The compounds of formula (I) according to the present invention can be prepared as described in WO-2017093093-A1.

In a preferred embodiment, the present invention relates to the use of a compound of a formula (I) with all the definitions and preferences as given herein as MMP-3 inhibitor, in particular for the protection of collagen-IV, laminin and proteoglycans, in particular in the basement membrane.

Furthermore, the invention relates to the uses and methods as disclosed herein for the protection of collagen-IV, laminin and proteoglycans against age-related degenerations of said collagen-IV, laminin and proteoglycans in the basement membrane and/ or slowing down the decrease said collagen-IV, laminin and proteoglycans in the BM during ageing.

In a further embodiment, the invention relates to the uses and methods as disclosed herein for improving lax, rough, dry and wrinkled skin caused by endogenous and exogenous degeneration of the basal membrane.

In another embodiment, the invention relates to the uses and methods as disclosed herein for slowing down and/ or preventing the formation of lax, rough, dry and wrinkled skin due to endogenous and exogenous degeneration of the basal membrane.

In another embodiment the present invention relates to the use of a compound of formula (I) with all the definitions and preferences as given herein for accelerating the healing of a wound.

It is particular preferred that the uses and methods according to the present invention are a cosmetic uses, i.e. for the cosmetic treatment of the human skin (to beautify the skin).

The term 'cosmetic composition' as used herein refers to compositions, which are used to treat, care for or improve the appearance of the skin and/or the scalp. Particular advantageous cosmetic compositions are skin care compositions.

The cosmetic compositions according to the invention are preferably intended for topical application, which is to be understood as the external application to keratinous substances, such as in particular the skin.

The term 'cosmetically acceptable carrier' as used herein refers to a physiologically acceptable medium which is compatible with keratinous substances. Suitable carriers are well known in the art and are selected based on the end-use application. Preferably, the carriers of the present invention are suitable for application to skin (e.g., sunscreens, creams, milks, lotions, masks, serums, hydrodispersions, foundations, creams, creamgels, or gels etc.). Such carriers are well known to one of ordinary skill in the art, and can include one or more compatible liquid or solid filler diluent, excipient, additive or vehicle, which are suitable for application to skin. The exact amount of carrier will depend upon the level of the compound of formula (I) and any other optional ingredients that one of ordinary skill in the art would classify as distinct from the carrier (e.g., other active components). The compositions of the present invention preferably comprise from about 75% to about 99.999%, more preferably from about 85% to about 99.99%, still more preferably from 90% to about 99%, and most preferably, from about 93% to about 98%, by weight of the composition, of a carrier.

The cosmetic compositions of the present invention can be formulated into a wide variety of product types, including creams, waxes, pastes, lotions, milks, mousses, gels, oils, tonics, and sprays. Preferably the compounds of formula (I) are formulated into lotions, creams, gels, and sprays. These product forms may be used for a number of applications, including, but not limited to, hand and body lotions, facial moisturizers, anti-ageing preparations, make-ups including foundations, and the like. Any additional components required to formulate such products vary with product type and can be routinely chosen by one skilled in the art.

If compositions of the present invention are formulated as an aerosol and applied to the skin as a spray-on product, a propellant is added to the composition.

The amount of the compound of formula (I) in the cosmetic composition can easily be adjusted by a person skilled in the art in order to achieve the desired beneficial effect. Preferably, the amount of the compound of formula (I) in the cosmetic compositions according to the present invention is at least 1 ppm based on the total weight of the cosmetic composition. In all embodiments of the present invention the amount of the compound of formula (I) is preferably selected in the range of about 0.00001 to 0.5 wt.-%, more preferably in the range of 0.0001 to 0.25 wt.-%, most preferably in the range of 0.0001 to 0.1 wt.-% based on the total weight of the cosmetic composition.

The cosmetic compositions according to the present invention can be prepared by conventional methods in the art such as e.g. by admixing a compound of formula (I) with all the definitions and preferences given herein with the cosmetically acceptable carrier.

The cosmetic compositions of the invention (including the carrier) may comprise further conventional cosmetic adjuvants and additives, such as preservatives/antioxidants, fatty substances/oils, water, organic solvents, silicones, thickeners, softeners, emulsifiers, antifoaming agents, aesthetic components such as fragrances, surfactants, fillers, anionic, cationic, nonionic or amphoteric polymers or mixtures thereof, propellants, acidifying or basifying agents, dyes, colorings/colorants, abrasives, absorbents, chelating agents and/ or sequestering agents, essential oils, skin sensates, astringents, pigments or any other ingredients usually formulated into such compositions.

In accordance with the present invention, the cosmetic compositions according to the invention may also comprise further cosmetically active ingredients conventionally used in cosmetic compositions. Exemplary active ingredients encompass skin lightening agents; UV-filters, agents for the treatment of hyperpigmentation; agents for the prevention or reduction of inflammation; firming, moisturizing, soothing, and/ or energizing agents as well as agents to improve elasticity and skin barrier.

Examples of cosmetic excipients, diluents, adjuvants, additives as well as active ingredients commonly used in the skin care industry which are suitable for use in the cosmetic compositions of the present invention are for example described in the International Cosmetic Ingredient Dictionary & Handbook by Personal Care Product Council (http://www.personalcarecouncil.org/), accessible by the online INFO BASE (http://online.personalcarecouncil.org/jsp/Home.jsp), without being limited thereto.

The necessary amounts of the active ingredients as well as the cosmetic excipients, diluents, adjuvants, additives etc. can, based on the desired product form and application, easily be determined by the skilled person. The additional ingredients can either be added to the oily phase, the aqueous phase or separately as deemed appropriate.

The cosmetically active ingredients useful herein can in some instances provide more than one benefit or operate via more than one mode of action.

Of course, one skilled in this art will take care to select the above mentioned optional additional ingredients, adjuvants, diluents and additives and/or their amounts such that the advantageous properties intrinsically associated with the combination in accordance with the invention are not, or not substantially, detrimentally affected by the envisaged addition or additions.

The cosmetic compositions according to the present invention may be in the form of a suspension or dispersion in solvents or fatty substances, or alternatively in the form of an emulsion or micro emulsion, in particular of oil-in-water (O/W) or water-in-oil (W/O) type, silicone-in-water (Si/W) or water-in-silicone (W/Si) type, PIT-emulsion, multiple emulsion (e.g. oil-in-water-in oil (O/W/O) or water-in-oil-in-water (W/O/W) type), pickering emulsion, hydrogel, alcoholic gel, lipogel, one- or multiphase solution or vesicular dispersion or other usual forms, which can also be applied by pens, as masks or as sprays.

If the cosmetic composition is an emulsion, such as in particular an O/W, W/O, Si/W, W/Si, O/W/O, W/O/W multiple or a pickering emulsion, then the amount of the oily phase present in such cosmetic emulsions is preferably at least 10 wt.-%, such as in the range of 10 to 60 wt.-%, preferably in the range of 15 to 50 wt.-%, most preferably in the range of 15 to 40 wt.-%, based on the total weight of the cosmetic composition.

In one embodiment, the cosmetic compositions according to the present invention are advantageously in the form of an oil-in-water (O/W) emulsion comprising an oily phase dispersed in an aqueous phase in the presence of an O/W emulsifier. The preparation of such O/W emulsions is well known to a person skilled in the art.

If the cosmetic composition according to the invention is an O/W emulsion, then it contains advantageously at least one O/W- or Si/W-emulsifier selected from the list of, glyceryl stearate citrate, glyceryl stearate SE (self-emulsifying), stearic acid, salts of stearic acid, polyglyceryl-3-methylglycosedistearate. Further suitable emulsifiers are phosphate esters and the salts thereof such as cetyl phosphate (e.g. as Amphisol® A from DSM Nutritional Products Ltd.), diethanolamine cetyl phosphate (e.g. as Amphisol® DEA from DSM Nutritional Products Ltd.), potassium cetyl phosphate (e.g. as Amphisol® K from DSM Nutritional Products Ltd.), sodium cetearylsulfate, sodium glyceryl oleate phosphate, hydrogenated vegetable glycerides phosphate and mixtures thereof. Further suitable emulsifiers are sorbitan oleate, sorbitan sesquioleate, sorbitan isostearate, sorbitan trioleate, cetearyl glucoside, lauryl glucoside, decyl glucoside, sodium stearoyl glutamate, sucrose polystearate and hydrated polyisobutene. Furthermore, one or more synthetic polymers may be used as an emulsifier. For example, PVP eicosene copolymer, acrylates/C10-30 alkyl acrylate crosspolymer, and mixtures thereof.

The at least one O/W, respectively Si/W emulsifier is preferably used in an amount of 0.5 to 10 wt. %, in particular in the range of 0.5 to 6 wt.-%, such as more in particular in the range of 0.5 to 5 wt.-%, such as most in particular in the range of 1 to 4 wt.-%, based on the total weight of the cosmetic composition.

Particular suitable O/W emulsifiers to be used in the cosmetic compositions according to the invention encompass phosphate ester emulsifiers such as advantageously 8-10 alkyl ethyl phosphate, C9-15 alkyl phosphate, ceteareth-2 phosphate, ceteareth-5 phosphate, ceteth-8 phosphate, ceteth-10 phosphate, cetyl phosphate, C6-10 pareth-4 phosphate, C12-15 pareth-2 phosphate, C12-15 pareth-3 phosphate, DEA-ceteareth-2 phosphate, DEA-cetyl phosphate, DEA-oleth-3 phosphate, potassium cetyl phosphate, deceth-4 phosphate, deceth-6 phosphate and trilaureth-4 phosphate.

A particular suitable O/W emulsifier to be used in the cosmetic compositions according to the invention is potassium cetyl phosphate e.g. commercially available as Amphisol® K at DSM Nutritional Products Ltd Kaiseraugst.

Another particular suitable class of O/W emulsifiers are non-ionic self-emulsifying systems derived from olive oil e.g. known as (INCI Name) cetearyl olivate and sorbitan olivate (chemical composition: sorbitan ester and cetearyl ester of olive oil fatty acids) sold under the tradename OLIVEM 1000.

In one particular embodiment, the invention relates to cosmetic compositions with all the definitions and preferences given herein in the form of O/W emulsions comprising an oily phase dispersed in an aqueous phase in the presence of an O/W emulsifier wherein the O/W emulsifier is potassium cetyl phosphate. The amount of oily phase in such O/W emulsions is preferably at least 10 wt.-%, more preferably in the range of 10 to 60 wt.-%, most preferably in the range of 15 to 50 wt.-%, such as in the range of 15 to 40 wt.-%.

The cosmetic compositions according to the invention in general have a pH in the range of 3 to 10, preferably a pH in the range of 4 to 8 and most preferably a pH in the range of 4 to 7.5. The pH can easily be adjusted as desired with suitable acids, such as e.g. citric acid, or bases, such as sodium hydroxide (e.g. as aqueous solution), triethanolamine (TEA Care), tromethamine (Trizma Base) and aminomethylpPropanol (AMP-Ultra PC 2000), according to standard methods in the art.

The amount of the cosmetic composition to be applied to the skin is not critical and can easily be adjusted by a person skilled in the art. Preferably, the amount is selected in the range of 0.1 to 3 mg/cm² skin, such as preferably in the range of 0.1 to 2 mg/cm² skin and most preferably in the range of 0.5 to 2 mg/cm² skin.

Further suitable uses of the compounds according to the present invention encompass pharmaceutical applications. Thus, the compounds according to the present invention may be used to prepare a pharmaceutical composition together with a pharmaceutical acceptable carrier, diluent or excipient for the treatment, prevention and/or prophylaxis of any disorder and disease where it is desirable to inhibit MMP-12 in a patient in need thereof such as e.g. for the treatment, prevention and/or prophylaxis of cancer, rheumatoid arthritis, osteoarthritis.

The invention is further illustrated with reference to the following, non-limiting examples, in which all percentages are by weight based on total weight unless otherwise specified.

### Experimental part

The MMP-3 inhibitor activity was quantified by fluorometric analysis using the MMP-3 Fluorometric Drug Discovery Kit (Enzo Life Sciences Inc., Farmingdale, NY, USA) according to the manufacturers' instructions). Briefly, the enzyme diluted in assay buffer was preincubated with several concentrations of potential inhibitors at 37°C for 30 minutes. Afterwards, a defined concentration of substrate was added, and its conversion was detected every minute for 30 minutes using a fluorescent microplate reader (Ex/Em=328/420). To obtain the reaction velocity v (RFU/min), the slope in the linear region of the reaction was calculated for each sample. The remaining MMP3 activity after inhibitor incubation was then calculated as follows: (v inhibitor / v control) x 100. These values were plotted in an x-y diagram and the data points were connected with a line. The IC50 was estimated by intersecting this line with the 50% activity mark.

**Table 2**

| # | Structure | IC50 |
|---|---|---|
| Inv-1 | 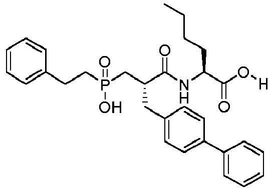 | 22 |
| | Ph-CH₂CH₂-P(O)(OH)-CH₂-CH(CH₂-Bip)-C(O)Nle-OH (L / L) | |
| Inv-2 | 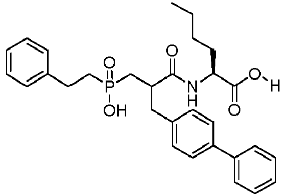 | 41 |
| | Ph-CH₂CH₂-P(O)(OH)-CH₂-CH(CH₂-Bip)-C(O)-Nle-OH (R,L / L) (isomeric mixture) | |
| Inv-3 | 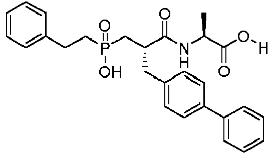 | 48 |
| | Ph-CH₂CH₂-P(O)(OH)-CH₂-CH(CH₂-Bip)-C(O)-Ala-OH (L / L) | |
| Inv-4 | 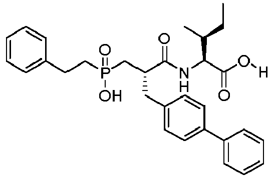 | 20 |
| | Ph-CH₂CH₂-P(O)(OH)-CH₂-CH(CH₂-Bip)-C(O)-Ile-OH (L / L) | |
| Inv-5 | 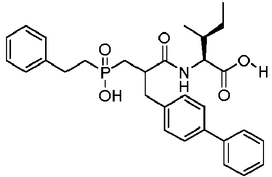 | 53 |
| | Ph-CH₂CH₂-P(O)(OH)-CH₂-CH(CH₂-Bip)-C(O)-Ile-OH (R,L / L) (isomeric mixture) | |
| Ref-1 | 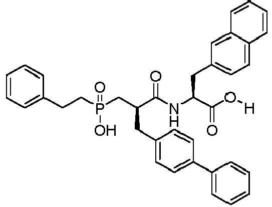 | 180 |
| | Ph-CH₂CH₂-P(O)(OH)-CH₂-CH(CH₂-Bip)-C(O)-2Nal-OH (R / L) | |
| Ref-2 | 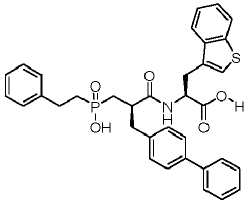 | 650 |
| | Ph-CH₂CH₂-P(O)(OH)-CH₂-CH(CH₂-Bip)-C(O)-Bta-OH (R / L) | |
| Ref-3 | 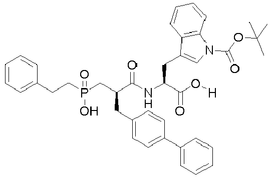 | 850 |
| | Ph-CH₂CH₂-P(O)(OH)-CH₂-CH(CH₂-Bip)-C(O)-Trp(Boc)-OH (R / L) | |
| Ref-4 | 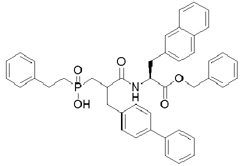 | 1500 |
| | Ph-CH₂CH₂-P(O)(OH)-CH₂-CH(CH₂-Bip)-C(O)-2Nal-OBn (L,R / L) (isomeric mixture) | |
| Ref-5 | 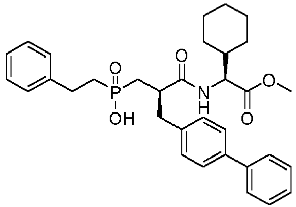 | 2200 |
| | Ph-CH₂CH₂-P(O)(OH)-CH₂-CH(CH₂-Bip)-Chg-OMe (R / L) | |

As can be retrieved from the results outlined in table 2, only the compounds according to the present invention are highly effective MMP-3 inhibitors, while e.g. the respective esters show a significant lower activity.

## Claims

1. A non-therapeutic use of a compound of formula (I) 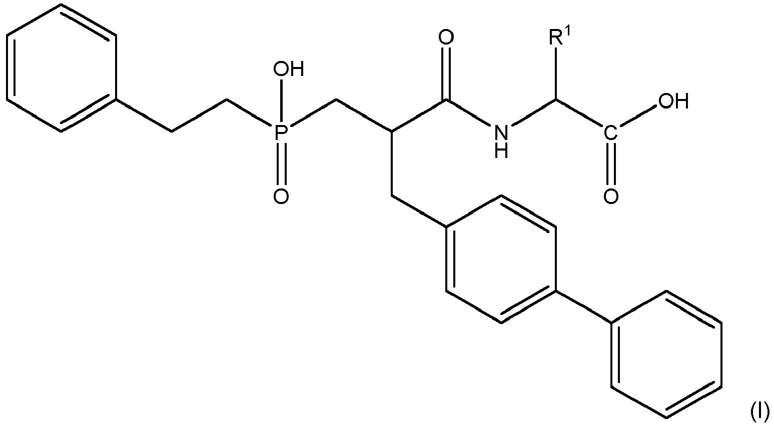 wherein
R¹ is a linear or branched C₁-C₁₀alkyl group
or a cosmetically acceptable salt thereof
as cosmetic active ingredient for inhibiting the degradation of the molecules of the basement membrane

2. The use according to claim 1, wherein said molecules are proteins and proteoglycans of the basement membrane.

3. The use according to claim 2, wherein said proteins are collagen IV and laminin.

4. The use according to any one of claims 1 to 3, for improving lax, rough, dry and wrinkled skin caused by endogenous and exogenous degeneration of the basal membrane.

5. The use according to any one of claims 1 to 3 for slowing down or preventing the formation of lax, rough, dry and wrinkled skin due to endogenous and exogenous degeneration of the basal membrane.

6. The use according to any one of claims 1 to 5,wherein R¹ is a linear or branched C₁-C₁₀alkyl group, preferably a linear or branched C₁₋₄alkyl group, most preferably methyl, butyl or sec-butyl.

7. A non-therapeutic method of inhibiting the degradation of molecules of the basement membrane, which method comprises topically administering to a person in need of such inhibition a composition containing an effective amount of at least one compound of formula (I) 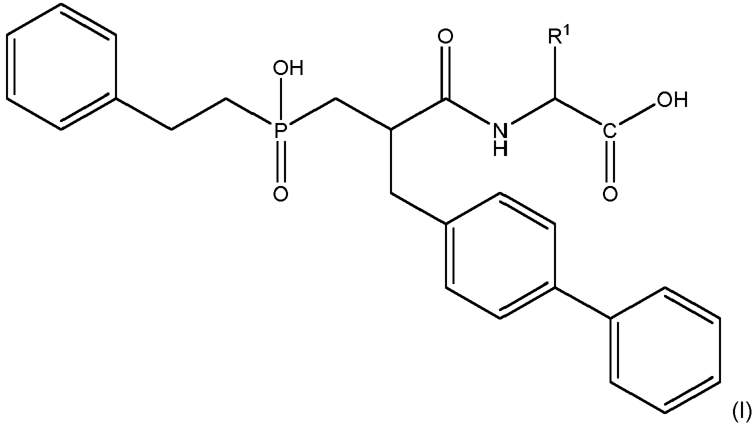 wherein R¹ is a linear or branched C₁-C₁₀alkyl group,
or a cosmetically acceptable salt thereof.

8. The method according to claim 7, wherein said molecules are proteins or proteoglycans of the basement membrane.

9. The method according to claim 8, wherein said proteins are collagen IV and/ or laminin.

10. The method according to any one of claims 7 to 9, for improving lax, rough, dry and wrinkled skin caused by endogenous and exogenous degeneration of the basal membrane.

11. The method according to any one of claims 7 to 9, for slowing down or preventing the formation of lax, rough, dry and wrinkled skin due to endogenous and exogenous degeneration of the basal membrane.

12. The method according to any one of claim 7 to 9, wherein R¹ is a linear or branched C₁-C₁₀alkyl group, preferably a linear or branched C₁₋₄alkyl group, most preferably methyl, butyl or sec-butyl.

13. A compound according of formula (I) 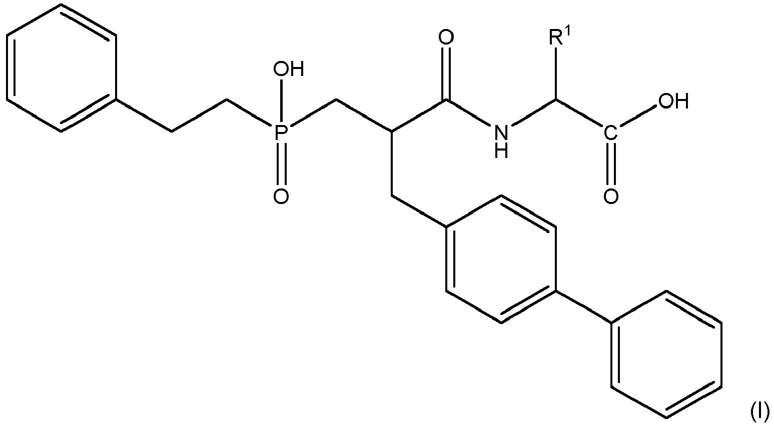 wherein R¹ is a linear or branched C₁-C₁₀alkyl group
or a cosmetically acceptable salt thereof for the use as MMP-3 inhibitor.

14. The use according to claim 13, wherein R¹ is a linear or branched C₁-C₁₀alkyl group, preferably a linear or branched C₁₋₄alkyl group, most preferably methyl, butyl or sec-butyl.
